(19) Europäisches Patentamt
European Patent Office
Office européen des brevets

(11) Publication number: **0 253 360 B1**

(12) **EUROPEAN PATENT SPECIFICATION**

(45) Date of publication of patent specification: **01.12.93**

(51) Int. Cl.[5]: **C07D 213/84**, C07D 241/24

(21) Application number: **87110158.0**

(22) Date of filing: **14.07.87**

(54) **Process for preparing nitriles.**

(30) Priority: **15.07.86 JP 165767/86**
**04.08.86 JP 183255/86**
**16.09.86 JP 218485/86**

(43) Date of publication of application:
**20.01.88 Bulletin 88/03**

(45) Publication of the grant of the patent:
**01.12.93 Bulletin 93/48**

(84) Designated Contracting States:
**BE DE IT**

(56) References cited:
**EP-A- 0 037 123**
**DE-B- 2 039 497**
**GB-A- 2 093 722**
**US-A- 4 603 207**
**DD-A- 241 903**
**DE-B- 2 741 625**
**US-A- 2 861 999**

**PATENT ABSTRACTS OF JAPAN, vol. 2, no. 155, 26th December 1978, page 3769C78 ; & JP-A-53121738**

(73) Proprietor: **KOEI CHEMICAL CO., LTD.**
**7, Yokobori-2-Chome**
**Higashi-ku**
**Osaka(JP)**

(72) Inventor: **Shimizu, Shinkichi**
**3-18-14, Fujisaka Motomachi**
**Hirakata-shi Osaka-fu(JP)**
Inventor: **Shoji, Takayuki**
**4-14-13, Sumiyoshi-ku**
**Osaka-shi Osaka-fu(JP)**
Inventor: **Abe, Nobuyuki**
**2-9-18, Asukano Minami**
**Ikoma-shi nara-ken(JP)**
Inventor: **Doba, Masanori**
**8-23-707, Nankoh Naka 3-chome**
**Suminoe-ku**
**Osaka-shi Osaka-fu(JP)**
Inventor: **Taguro, Akira**
**2-25-506, Otokoyama Yumioka**
**Yahata-shi Kyoto-fu(JP)**
Inventor: **Iguchi, Akira**
**2-12-13, Hanaten Nishi**
**Johto-ku**
**Osaka-shi Osaka-fu(JP)**
Inventor: **Nakaishi, Toru**
**2-12-13, Hanaten Nishi**
**Johto-ku**
**Osaka-shi Osaka-fu(JP)**

(74) Representative: **VOSSIUS & PARTNER**
**Postfach 86 07 67**
**D-81634 München (DE)**

**EP 0 253 360 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing nitriles. More particularly, it relates to a process for producing a heteroaromatic nitrile comprising catalytically reacting at least one compound selected from the group consisting of alkyl-substituted pyridines and alkyl-substituted pyrazines with ammonia and molecular oxygen in a gaseous phase (namely, by ammoxidation).

The heteroaromatic nitriles are useful as starting materials for the preparation of medicines or agricultural chemicals.

For catalyzing ammoxidation of an alkyl-substituted aromatic compound to prepare an aromatic nitrile, a number of catalysts comprising vanadium oxide are proposed. However, the conventional catalysts have too strong catalytic activity for the ammoxidation of alkyl-substituted heteroaromatic compounds, abnormal reactions such as dealkylation or cleavage of the heteroaromatic ring and therefore the objective nitriles are prepared in a low selectivity and yield.

Japanese Patent Publication No. 19706/1982 discloses, as a catalyst for ammoxidation of the alkyl-substituted heteroaromatic compound, a catalyst comprising antimony oxide, vanadium oxide and an oxide of a metal selected from the group consisting of iron, copper, titanium, cobalt, manganese and nickel. Although this catalyst has a comparatively high selectivity, it suffers from decrease of catalytic activity through reduction with ammonia and is not satisfactory for industrial production.

In the patent abstract of Japan, vo. 2, No. 155 (1978), p. 3769C78, a process for the preparation of aromatic nitriles is disclosed. This document does not mention heteroaromatic compounds and does not disclose any oxygen to ammonia rates.

US-PS 2861999 discloses a process for the production of cyanopyridines with the use of an oxidation catalyst having a particle size of 50 to 3000 microns and containing oxides of vanadium, molybdenum and phosphorus supported on activated alumina.

UK-PS 2093722 discloses catalysts for the catalytic reaction of 2-methyl-pyrazine with ammonia and oxygen to produce 2-cyano-pyrazine. No air to ammonia ration is mentioned in UK-PS 2093722.

As a result of the extensive study, it has been found that, in the preparation of the heteroaromatic nitriles by ammoxidation of at least one compound selected from the group consisting of alkyl-substituted pyridines and alkyl-substituted pyrazines, the abnormal reactions can be suppressed and the heteroaromatic nitriles can be prepared in a high selectivity and yield when a molar ratio of molecular oxygen and ammonia is selected and a vanadium-phosphorus base compound is used as a catalyst, and further said catalyst has good resistance against heat and reduction and is safe in operation. The present invention is completed based on these findings.

According to the present invention, there is provided a process for preparing a heteroaromatic nitrile comprising catalytically reacting at least one compound selected from the group consisting of alkyl-substituted pyridines and alkyl-substituted pyrazines with molecular oxygen and ammonia in a molar ratio of air to ammonia of not larger than 11:2 in a gaseous phase in the presence of a catalyst comprising an oxide of the formula:

$$VP_xSb_yO_z \qquad (I)$$

wherein x, y and z represent atomic ratios of phosphorus, antimony and oxygen to vanadium, respectively, and x is from 0.1 to 5, y is 0 to 8 and z is defined from the valencies of other elements.

The catalyst to be used according to the present invention may be any one containing the oxide (I). A vanadium-phosphorus oxide, namely the oxide (I) wherein y is 0 (zero) can be used in the amorphous or crystalline form according to the present invention. When the vanadium-phosphorus oxide is amorphous, an atomic ratio of vanadium and phosphorus is not critical. Preferably, said ratio is from 1:0.5 to 1:3. As the crystalline vanadium-phosphorus oxide, one in which the atomic ratio of vanadium and phosphorus is 1, 2, 3 and the like is known. Among them, one in which the atomic ratio of vanadium and phosphorus is 1 and/or a reduction product of such vanadium-phosphorus oxide are effective in the process according to the present invention.

The crystalline oxide used herein is represented by the formula:

$$(VO)_nP_nO_{4n-1}.xH_2O \qquad (II)$$

wherein n is an integer not less than 2 and x is 0 or a positive integer. In the formula (II), the valency of vanadium is 4 or 5.

Specific examples of the compound (II) are alpha-vanadyl phosphate (alpha-$VOPO_4$), alpha-vanadyl phosphate dihydrate (alpha-$VOPO_4.2H_2O$), beta-vanadyl phosphate (beta-$VOPO_4$), vanadyl pyrophosphate ($(VO)_2P_2O_7$) and vanadyl pyrophosphate hydrate ($(VO)_2H_4P_2O_9$ or $(VO)_2P_2O_7.2H_2O$), X-ray diffraction patterns of which are shown in Table 1.

Table 1

| Alpha-$VOPO_4$ d(Å) | Alpha-$VOPO_4 \cdot 2H_2O$ d(Å) | Beta-$VOPO_4$ d(Å) | $(VO)_2P_2O_7$ d(Å) | $(VO)_2H_4P_2O_9$ d(Å) |
|---|---|---|---|---|
| 4.37 | 7.41 | 5.18 | 6.28 | 5.625 |
| 4.11 | 4.76 | 4.60 | 5.65 | 4.761 |
| 3.100 | 4.37 | 3.96 | 4.79 | 4.058 |
| 3.000 | 3.70 | 3.89 | 3.87 | 3.659 |
| 2.193 | 3.18 | 3.48 | 2.982 | 3.275 |
| 1.963 | 3.105 | 3.40 | 2.906 | 3.093 |
| 1.550 | 2.863 | 3.18 | 2.655 | 2.92 |
| 1.517 | 2.833 | 3.068 | 2.435 | 2.777 |
| 1.461 | 2.379 | 2.974 | 2.399 | |
| | 2.197 | 2.640 | 2.362 | |
| | 2.108 | 2.410 | 2.271 | |
| | | 2.209 | 2.204 | |
| | | 2.172 | 2.083 | |
| | | 2.093 | | |

A vanadium-phosphorus oxide prepared by the reduction of these compounds has generally less crystallinity. This may be due to formation of lattice defects or shear structures.

The vanadium-phosphorus oxide (the oxide (I) wherein y is 0) may be prepared by a per se know method for preparing an oxide of vanadium and phosphorus. For example, the amorphous vanadium-phosphorus oxide is prepared by reacting ammonium metavanadate dissolved in an aqueous solution of ethanolamine with phosphoric acid and evaporating a reaction mixture to dryness and calcining the dried product in the air. The crystalline vanadium-phosphorus oxide is prepared by reacting vanadium pentoxide with phosphoric acid, evaporating a reaction mixture to dryness and calcining the dried product in the air,

by reacting ammonium metavanadate with phosphoric acid, evaporating a reaction mixture to dryness and calcining the dried product in the air, or by reacting vanadium pentoxide, oxalic acid and ammonium dihydrogenphosphate, evaporating a reaction mixture to dryness and calcining the dried product in nitrogen.

A vanadium-phosphorus oxide prepared by reducing the crystalline oxide with a reducing gas such as hydrogen, ammonia or hydrocarbons may be used as a catalyst according to the present invention. For example, the crystalline oxide is preferably reduced by treating it with ammonia or an ammonia-containing gas at a temperature of 300 to 600 °C.

Among the oxide (I) wherein y is not 0 (zero), those wherein x is from 0.2 to 1.5 and y is from 2 to 4 are preferred. The oxide (I) wherein y is not 0 may also be prepared by a per se known method for preparing an oxide of vanadium and phosphorus. For example, the oxide is prepared by adding a vanadium compound, a phosphorus compound and an antimony compound and optionally a carrier to water, evaporating the mixture to dryness and calcining it at a temperature of 450 to 800 °C in the air or an inactive gas for several to 20 hours.

For the preparation of the catalyst, any compound of each element can be used. Specific examples of the vanadium compound are ammonium metavanadate, vanadium pentoxide, vanadium phosphate, etc. Specific examples of the phosphorus compound are phosphoric acid, metaphosphoric acid, phosphorous acid, phosphate (such as ammonium phosphates), etc. Specific examples of the antimony compound are metal antimony, diantimony trioxide, diantimony pentoxide, antimony trichloride, etc.

The catalyst used according to the present invention may be carried on a carrier such as silica, alumina, silicon carbide, titanium oxide, diatomaceous earth and zeolite.

The alkyl-substituted pyridines or pyrazines used according to the present invention are compounds of the formula:

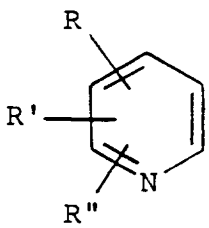 or 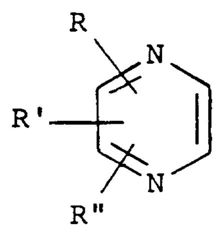

wherein R, R' and R" are the same or different and each a hydrogen atom or a lower alkyl group having 1 to 4 carbon atoms with the proviso that at least one of them is the alkyl group. Specific examples of the alkyl-substituted pyridines or pyrazines are 2-methylpyridine, 3-methylpyridine, 4-methylpyridine, 2,3-dimethylpyridine, 2,4-dimethylpyridine, 2,5-dimethylpyridine, 2,6-dimethylpyridine, 3,4-dimethylpyridine, 3,5-dimethylpyridine, 2-methyl-5-ethylpyridine, 2,4,6-trimethylpyridine, 2,3,4-trimethylpyridine, 2,3,5-trimethylpyridine, 2,3,6-trimethylpyridine, methylpyrazine, ethylpyrazine, 2,3-dimethylpyrazine, 2,5-dimethylpyrazine, 2,6-dimethylpyrazine, 2-methyl-5-ethylpyrazine, 2-methyl-6-ethylpyrazine, etc.

The concentration of the alkyl-substituted heteroaromatic compound in the gaseous reaction mixture may be from 0.15 to 7 % by mole. In the reaction mixture, the molar ratio of air and the ammonia is not larger than 11:2 When said molar ratio exceeds 11:2, undesirable reactions such as perfect combustion of the alkyl-substituted compound and ammonia occur. Thereby, the yield of the desired nitrite greatly decreases.

As molecular oxygen, air is preferably used although pure oxygen or a mixture of pure oxygen and air may be used.

The gaseous reaction mixture containing the alkyl-substituted pyridine or pyrazine, molecular oxygen and ammonia may be diluted with an inactive gas such as steam or nitrogen.

In the process according to the present invention, the reaction temperature is from 300 to 650 °C, preferably from 350 to 600 °C. The space velocity is from 200 to 50,000 $hr^{-1}$ preferably from 300 to 20,000 $hr^{-1}$. Usually, the reaction according to the present invention is carried out under atmospheric pressure, although it may be carried out under reduced or high pressure. The reaction of the present invention is generally performed with a fixed bed reactor, although it may be performed with a fluidized bed reactor.

The present invention will be hereinafter explained further in detail by following examples, in which a conversion and a yield are calculated by following equations:

$$\text{Convertion (\%)} = 100 \times \frac{\text{Reacted alkyl-substituted pyridine or pyrazine (mole)}}{\text{Supplied alkyl-substituted pyridine or pyrazine (mole)}}$$

$$\text{Yield (\%)} = 100 \times \frac{\text{Produced nitrile compound (mole)}}{\text{Supplied alkyl-substituted pyridine or pyrazine (mole)}}$$

## Example 1

In distilled water (1,250 g) kept at 60 °C, ammonium metavanadate (146.5 g) was dissolved with stirring and to the resulting solution, 85 % phosphoric acid (144 g) was added and reacted for 2 hours to obtain a reaction mixture containing a precipitated yellow compound. The reaction mixture was concentrated and dried at 110 °C for 8 hours followed by calcination at 500 °C for 4 hours in the air. The resulting catalyst was beta-vanadyl phosphate (beta-$VOPO_4$), X-ray diffraction pattern of which corresponded to that in Table 1.

The catalyst (10 ml) was filled in a Pyrex glass made reactor tube having an inner diameter of 12.6 mm. With heating the catalyst filled portion of the reactor at 420 °C, a gaseous mixture of 4-methylpyridine, ammonia, air and steam (molar ratio = 1:15:75:10) was flowed through the reactor at a space velocity of 3,000 $hr^{-1}$ and the reacted gaseous mixture was trapped by water for 20 minutes and analyzed by gas chromatography. Conversion of 4-methylpyridine, 99.4 %. Yield of 4-cyanopyridine, 81.5 %.

## Example 2

In the same manner as in Example 1 but flowing a gaseous mixture of 4-methylpyridine, ammonia, air and steam (molar ratio = 1:10:10:15) through the reactor tube kept at 400 °C at a space velocity of 2,000 $hr^{-1}$, the reaction was carried out. Conversion of 4-methylpyridine, 99.5 %. Yield of 4-cyanopyridine, 94.0 %.

## Comparative Example 1

In the same manner as in Example 1 but flowing a gaseous mixture of 4-methylpyridine, ammonia, air and steam (molar ratio = 1:2:75:10) through the reactor tube, the reaction was carried out. Conversion of 4-methylpyridine, 99.3 %. Yield of 4-cyanopyridine, 4.2 %.

## Comparative Example 2

In the same manner as in Example 1 but flowing a gaseous mixture of 4-methylpyridine, ammonia, air and steam (molar ratio = 1:8:75:10) through the reactor tube at a space velocity of 2,000 $hr^{-1}$, the reaction was carried out. Conversion of 4-methylpyridine, 99.4 %. Yield of 4-cyanopyridine, 9,6 %.

## Example 3

To 85 % phosphoric acid (63 g) heated to a temperature of 95 to 100 °C, vanadium pentoxide (50 g) was added to obtain a reaction mixture containing yellow precipitates of $VOPO_4.2H_2O$. The reaction mixture was concentrated and dried at 110 °C for 8 hours followed by calcination at 500 °C for 6 hours and at 700 °C for 4 hours in the air. The obtained catalyst was alpha-vanadyl phosphate (alpha-$VOPO_4$), X-ray diffraction pattern of which corresponded to that in Table 1.

The catalyst was mixed with silicon carbide in a volume ratio of 1:1. The mixture (10 ml) was then filled in a Pyrex glass made reactor tube having an inner diameter of 12.6 mm.

With heating the catalyst filled portion of the reactor at 450 °C, a gaseous mixture of 2-methylpyridine, ammonia, air and steam (molar ratio = 1:10:10:10) was flowed through the reactor at a space velocity of 3,000 $hr^{-1}$. After 10 hour reaction, the reacted gaseous mixture was analyzed by gas chromatography. Conversion of 2-methylpyridine, 93.8 %. Yield of 2-cyanopyridine, 76.3 %.

Example 4

To distilled water (600 ml), 85 % phosphoric acid (252.8 g) was added with stirring, and after heated to 95 °C, vanadium pentoxide (200 g) was added and reacted for 30 minutes. After adding silica (132 g), the reaction mixture was stirred for one hour and concentrated to obtain a reaction mixture containing a precipitated compound. The precipitated compound was dried at 110 °C for 8 hours followed by calcination at 500 °C for 6 hours and at 700 °C for 4 hours in the air. The resulting catalyst was beta-vanadyl phosphate/silica. X-ray diffraction pattern of beta-vanadyl phosphate (beta-$VOPO_4$) corresponded to that in Table 1.

The catalyst was filled in the reactor tube in the same manner as in Example 1. With heating the catalyst filled portion of the reactor at 430 °C, ammonia was flowed at a rate of 100 ml/min. for 20 hours to reduce the catalyst. Then, at the same temperature, a gaseous mixture of methylpyrazine, ammonia, air and steam (molar ratio = 1:20:11:1) was flowed through the reactor at a space velocity of 1,000 $hr^{-1}$ and the reacted gaseous mixture was analyzed by gas chromatography. Conversion of methylpyrazine, 94.8 %. Yield of cyanopyrazine, 81.5 %.

After 1,000 hour reaction, the conversion of methylpyrazine, and the yield of cyanopyrazine were 91.5 % and 81.2 %, respectively.

Example 5

In distilled water (750 g), oxalic acid (315 g) was dissolved with stirring and to the resulting solution heated at 80 °C, vanadium pentoxide (90.5 g) and then ammonium dihydrogenphosphate (115 g) were added followed by evaporation to dryness. The resulting solid was calcined at 550 °C for 5 hours in nitrogen to obtain vanadyl pyrophosphate ($(VO)_2P_2O_7$), X-ray diffraction pattern of which corresponded to that in Table 1.

In the same manner as in Example 2 but using this vanadyl pyrophosphate as a catalyst, 3-methylpyridine as a starting material and flowing the gaseous mixture at a space velocity of 1,000 $hr^{-1}$, the reaction was carried out. Conversion of 3-methylpyridine, 90.3 %. Yield of 3-cyanopyridine, 81.1 %.

Example 6

In a 10 % by weight aqueous solution of monoethanolamine (1,500 g), ammonium metavanadate (146.5 g) was dissolved with stirring and then 85 % phosphoric acid (144.0 g) was added and reacted for 30 minutes. The resulting solution was evaporated to dryness to obtain a solid compound, which was calcined in the air at 550 °C for 5 hours in the air to obtain a vanadium-phosphorus oxide ($VPO_5$), X-ray diffraction pattern of which showed that this oxide was amorphous.

In the same manner as in Example 2 but using this vanadium-phosphorus oxide as a catalyst and heating the reactor tube at 500 °C, the reaction was carried out. Convertion of 4-methylpyridine, 96.5 %. Yield of 4-cyanopyridine, 85.1 %.

After 500 hours from the start of the reaction, the convertion of 4-methylpyridine was 95.4 %, and the yield of 4-cyanopyridine was 84.7 %.

Examples 7-9

In the same manner as in Example 2 but using a catalyst having a composition as shown in Table 2 which had been prepared by the same manner as in Example 6, the reaction was carried out. The results are shown in Table 2.

Table 2

| Example No. | Catalyst | Conversion of 4-picoline (%) | Yield of 4-cyanopyridine (%) |
|---|---|---|---|
| 7 | $VP_{0.75}O_{4.375}$ | 99.8 | 80.2 |
| 8 | $VP_{1.2}O_{5.5}$ | 93.1 | 85.0 |
| 9 | $VP_{1.5}O_{6.25}$ | 91.3 | 83.4 |

Examples 10-13

By using beta-$VOPO_4/3SiO_2$ prepared by the same method as in Example 4, a reaction of 2-methylpyridine, 3-methylpyridine, 4-methylpyridine or methylpyrazine was carried out under the conditions specified in Table 3. The results are also shown in Table 3.

Table 3

| Example No. | Raw material | Molar ratio*1) | Space velocity ($hr^{-1}$) | Reactor temp. (%) | Conversion (%) | Yield of nitrile (%) |
|---|---|---|---|---|---|---|
| 10 | 2-Methyl-pyridine | 1/10/15/10 | 2,500 | 430 | 97.6 | 79.3 |
| 11 | 3-Methyl-pyridine | 1/2/11/13 | 1,800 | 420 | 96.1 | 82.3 |
| 12 | 4-Methyl-pyridine | 1/2/11/13 | 2,000 | 420 | 99.9 | 92.3 |
| 13 | Methyl-pyrazine | 1/25/15/2 | 1,200 | 430 | 98.6 | 83.5 |

Note: *1) Molar ratio of the raw material/ammonia/air/steam.

Example 14

By using the catalyst prepared in Example 4, a mixture of 65 % by mole of 3-methylpyridine and 35 % by mole of 4-methylpyridine as raw materials, the reaction was carried out at a reactor temperature of 430 °C, at a space velocity of 1,200 $hr^{-1}$ with a molar ratio of the raw materials, ammonia, air and steam shown in Table 4. The results are also shown in Table 4.

Table 4

| Molar ratio of raw materials/ammonia/air/steam | Conversion (%) | | Yield (%) | |
|---|---|---|---|---|
| | 3-Methylpyridine | 4-Methylpyridine | 3-Cyanopyridine | 4-Cyanopyridine |
| 1/5/10/15 | 98.7 | 100 | 79.9 | 90.1 |
| 1/5/4/15 | 51.5 | 100 | 37.3 | 90.3 |

Example 15

In water (0.5 l), ammonium metavanadate (24 g) and antimony trioxide (60 g) were suspended. To the resulting suspension, 85 % phosphoric acid (23.8 g) was added and heated and then silica (74 g) as a carrier was added and further heated to concentrate the suspension to obtain a paste, which was evaporated to dryness. The residue was calcined at 750 °C for 3 hours in the air. The obtained catalyst had a composition of $VSb_2P_{0.85}O_{7.62}$.

The catalyst (25 ml) was filled in the reactor tube in the same manner as in Example 1. The reactor tube was then heated at 420 °C.

A gaseous mixture of methylpyrazine, ammonia, air and steam in a molar ratio of 1:19:9:5 was flowed through the reactor at a space velocity of 850 $hr^{-1}$. Conversion of methylpyrazine, 99 %. Yield of cyanopyrazine, 86 %.

Example 16

In the same manner as in Example 15 but flowing a gaseous mixture of 2-methylpyridine, ammonia, air and steam in a molar ratio of 1:10:15:15 at a space velocity of 3,000 $hr^{-1}$, the reaction was carried out. Conversion of 2-methylpyridine, 99 %. Yield of 2-cyanopyridine, 73 %.

Example 17

In the same manner as in Example 15 but flowing a gaseous mixture of 4-methylpyridine, ammonia, air and steam in a molar ratio of 1:5:10:9 at a reactor temperature of 340 °C, at a space velocity of 3,000 $hr^{-1}$, the reaction was carried out. Conversion of 4-methylpyridine, 99 %. Yield of 4-cyanopyridine, 96 %.

After 500 hours, the yield was 92.3 %.

Example 18

In the same manner as in Example 4 but flowing a gaseous mixture of 3,5-dimethylpyridine, ammonia and air in a molar ratio of 1:40:40 at a reactor temperature of 420 °C, at a space velocity of 1,500 $hr^{-1}$, the reaction was carried out. Conversion of 3,5-dimethylpyridine, 100 %. Yield of 5-cyano-3-methylpyridine, 32.5 %. Yield of 3,5-dicyanopyridine, 44.0 %.

## Claims

1. A process for preparing a heteroaromatic nitrile comprising catalytically reacting at least one compound selected from the group consisting of alkyl-substituted pyridines and alkyl-substituted pyrazines with molecular oxygen and ammonia in a molar ratio which is not larger than a molar ratio calculated on the basis of an air to ammonia ratio of 11 : 2 in a gaseous phase in the presence of a catalyst comprising an oxide of the formula:

$$VP_xSb_yO_z \qquad (I)$$

wherein x, y and z represent atomic ratios of phosphorus, antimony and oxygen to vanadium, respectively, and x is from 0.1 to 5, y is 0 to 8 and z is defined from the valencies of other elements.

2. The process according to claim 1, wherein the oxide is a crystalline vanadium-phosphorus oxide of the formula (I) wherein y is 0 (zero) and the atomic ratio of vanadium and phosphorus is 1, or a reduction product of said vanadium-phosphorus oxide.

3. The process according to claim 2, wherein the crystalline oxide is an oxide of the formula:

$(VO)_nP_nO_{4n-1}.xH_2O$ (II)

wherein n is an integer not less than 2 and x is 0 or a positive integer.

4. The process according to claim 2, wherein the crystalline vanadium-phosphorus oxide is at least one oxide selected from the group consisting of alpha-vanadyl phosphate (alpha-$VOPO_4$), alpha-vanadyl phosphate dihydrate (alpha-$VOPO_4.2H_2O$), beta-vanadyl phosphate (beta-$VOPO_4$), vanadyl pyrophosphate ($(VO)_2P_2O_7$) and vanadyl pyrophosphate hydrate ($(VO)_2H_4P_2O_9$ or $(VO)_2P_2O_7.2H_2O$).

5. The process according to claim 1, wherein the catalyst is carried on a carrier selected from the group consisting of silica, alumina, silicon carbide, titanium oxide, diatomaceous earth and zeolite.

6. The process according to claim 2, wherein the catalyst is carried on a carrier selected from the group consisting of silica, alumina, silicon carbide, titanium oxide, diatomaceous earth and zeolite.

7. The process according to claim 2, wherein the reduction product of said vanadium-phosphorus oxide is a reduction product prepared by reducing said vanadium-phosphorus oxide with ammonia or an ammonia-containing gas at a temperature of 300 to 600 °C.

**Patentansprüche**

1. Verfahren zur Herstellung eines heteroaromatischen Nitrils, umfassend die katalytische Umsetzung mindestens einer Verbindung aus der Gruppe alkylsubstituierter Pyridine und alkylsubstituierter Pyrazine mit molekularem Sauerstoff und Ammoniak in einem Molverhältnis, das nicht größer ist als ein Molverhältnis, das auf der Basis eines Luft-Ammoniak-Verhältnisses von 11 : 2 berechnet ist, in der Gasphase in Gegenwart eines Katalysators, der ein Oxid der Formel

$VP_xSb_yO_z$ (I)

umfaßt, in der x, y und z Atomverhältnisse von Phosphor, Antimon bzw. Sauerstoff zu Vanadium darstellen, und x im Bereich von 0,1 bis 5 liegt, y 0 oder 8 bedeutet und z durch die Wertigkeiten der anderen Elemente definiert ist.

2. Verfahren nach Anspruch 1, in dem das Oxid ein kristallines Vanadiumphosphoroxid der Formel (I), in der y 0 (Null) ist und das Atomverhältnis von Vanadium und Phosphor 1 ist, oder ein Reduktionsprodukt dieses Vanadiumphosphoroxids ist.

3. Verfahren nach Anspruch 2, in dem das kristalline Oxid ein Oxid der Formel

$(VO)_nP_n O_{4n-1} \cdot xH_2O$ (II)

ist, in dem n eine ganze Zahl nicht kleiner als 2 ist und x 0 oder eine positive ganze Zahl bedeutet.

4. Verfahren nach Anspruch 2, in dem das kristalline Vanadiumphosphoroxid mindestens ein Oxid aus der Gruppe $\alpha$-Vanadylphosphat ($\alpha$-$VOPO_4$), $\alpha$-Vanadylphosphatdihydrat ($\alpha$-$VOPO_4 \cdot 2H_2O$); $\beta$-Vanadylphosphat ($\beta$-$VOPO_4$), Vanadylpyrophosphat ($(VO)_2P_2O_7$) und Vanadylpyrophosphathydrat ($(VO)_2H_4P_2O_9$ oder $(VO)_2P_2O_7 \cdot 2H_2O$) ist.

5. Verfahren nach Anspruch 1, in dem der Katalysator auf einen Träger aufgebracht ist, der aus der Gruppe Siliciumdioxid, Aluminiumoxid, Siliciumcarbid, Titanoxid, Diatomeenerde und Zeolit ausgewählt ist.

6. Verfahren nach Anspruch 2, in dem der Katalysator auf einen Träger aufgebracht ist, der aus der Gruppe Siliciumdioxid, Aluminiumoxid, Siliciumcarbid, Titanoxid, Diatomeenerde und Zeolit ausgewählt ist.

7. Verfahren nach Anspruch 2, in dem das Reduktionsprodukt des Vanadiumphosphoroxid ein Reduktionsprodukt ist, das durch Reduktion des Vanadiumphosphoroxids mit Ammoniak oder einem Ammoniak enthaltenden Gas bei einer Temperatur von 300 bis 600 °C hergestellt wird.

**Revendications**

1. Procédé de préparation d'un nitrile hétéroaromatique caractérisé en ce que l'on fait catalytiquement réagir au moins un composé choisi dans le groupe formé par des pyridines alkyl-substituées et des pyrazines alkyl-substituées avec de l'oxygène moléculaire et de l'ammoniac dans un rapport molaire qui n'est pas plus grand qu'un rapport molaire calculé sur la base d'un rapport air à ammoniac de 11:2, en phase gazeuse et en présence d'un catalyseur comprenant un oxyde de la formule :

   $VP_xSb_yO_z$ (I)

   dans laquelle x, y et z représentent les rapports atomiques du phosphore, de l'antimoine et de l'oxygène au vanadium respectivement et x a une valeur qui varie de 0,1 à 5, y varie de 0 à 8 et Z est défini à partir des valences des autres éléments.

2. Procédé suivant la revendication 1, caractérisé en ce que l'oxyde est un oxyde de vanadium-phosphore cristallin de la formule (I) dans laquelle y est égal à 0 (zéro) et le rapport atomique du vanadium et du phosphore est de 1, ou un produit de réduction dudit oxyde de vanadium-phosphore.

3. Procédé suivant la revendication 2, caractérisé en ce que l'oxyde cristallin est un oxyde de la formule :

   $(VO)_nP_nO_{4n-1}.xH_20$ (II)

   dans laquelle n est un nombre entier non inférieur à 2 et x est égal à 0 ou est un nombre entier positif.

4. Procédé suivant la revendication 2, caractérisé en ce que l'oxyde de vanadium-phosphore cristallin est au moins un oxyde choisi dans le groupe formé par le phosphate d'alpha-vanadyle (alpha-$VOPO_4$), le phosphate d'alpha-vanadyle dihydraté (alpha-$VOPO_4.2H_2O$), le phosphate de bêta-vanadyle (bêta-$VOPO_4$), le pyrophosphate de vanadyle ($(VO)_2P_2O_7$) et le pyrophosphate de vanadyle hydraté ($(VO)_2H_4P_2O_9$ ou $(VO)_2P_2O_7.2H_2O$).

5. Procédé suivant la revendication 1, caractérisé en ce que le catalyseur est porté par un support choisi dans le groupe formé par la silice, l'alumine, le carbure de silicium, l'oxyde de titane, la terre à diatomées et une zéolite.

6. Procédé suivant la revendication 2, caractérisé en ce que le catalyseur est porté par un support choisi dans le groupe formé par la silice, l'alumine, le carbure de silicium, l'oxyde de titane, la terre à diatomées et une zéolite.

7. Procédé suivant la revendication 2, caractérisé en ce que le produit de réduction dudit oxyde de vanadiumphosphore est un produit de réduction préparé par la réduction dudit oxyde de vanadium-phosphore avec de l'ammoniac ou un gaz contenant de l'ammoniac, à une température de 300 à 600 °C.